# EUROPÄISCHE PATENTSCHRIFT

(11) **EP 0 555 246 B1**
(45) Veröffentlichungstag und Bekanntmachung des Hinweises auf die Patenterteilung: **21.06.1995**
(21) Anmeldenummer: 91918050.5
(22) Anmeldetag: 21.10.1991
(51) Int. Cl.: C01F 7/00, C07C 41/03, C07C 53/126, C01B 13/14

(54) **HYDROPHOBIERTE DOPPELSCHICHTHYDROXID-VERBINDUNGEN**
HYDROPHOBISED DOUBLE-LAYER HYDROXIDE COMPOUNDS
COMPOSES HYDROXYDES IMPERMEABILISES A DOUBLE COUCHE

(30) Priorität: 29.10.1990 DE 4034305
(43) Veröffentlichungstag der Anmeldung: 18.08.1993
(73) Patentinhaber: Henkel Kommanditgesellschaft auf Aktien, 40191 Düsseldorf (DE)
(72) Erfinder: BREUER, Wolfgang, D-4000 Düsseldorf 1 (DE); RATHS, Hans-Christian, D-4019 Monheim 2 (DE)
(86) Internationale Anmeldenummer: EP9101993
(87) Internationale Veröffentlichungsnummer: WO9207795

(56) Entgegenhaltungen:
- EP-A- 0 095 783
- EP-A- 0 169 357
- EP-A- 0 339 426
- DE-A- 3 833 076
- US-A- 4 351 814

## Beschreibung

### Gegenstand der Erfindung

Die Erfindung betrifft neue hydrophobierte Doppelschichthyroxid-Verbindungen, ein Verfahren zur Herstellung dieser Verbindungen durch Umsetzung von Doppelschichthydroxiden mit aliphatischen Mono- und/oder Dicarbonsäuren in einem organichen Lösungsmittel oder in einem Kneter sowie die Verwendung der hydrophobierten Doppelschichthydroxid-Verbindungen als Alkoxylierungskatalysatoren für Verbindungen mit aktiven H-Atomen oder für Fettsäureester.

### Stand der Technik

Zwei-dimensionale anorganische Polykationen mit innerkristallinem Ladungsausgleich durch bewegliche Zwischenschichtanionen sind auch unter der Bezeichnung "Doppelschichthydroxid-Verindungen" bekannt und mehrfach in der Literatur beschrieben [ **himia 24, 99 (1970)**]. Chemisch stellen diese Verbindungen gemischte Hydroxosalze 2- und 3-wertiger Metallionen dar und lassen sich durch die allgemeine Formel charakterisieren:

**[M(II)**_{**1-x**}**M(III)**_{**x**}**(OH)₂]B * n H₂O**

wobei
- M(II): für mindestens ein 2-wertiges Metallion,
- M(III): für mindestens ein 3-wertiges Metallion und
- B: für ein Äquivalent einer ein- und/oder mehrbasischen, anorganischen Säure
stehen und
- x: eine Zahl von 0,2 bis 0,4 sowie
- n: eine Zahl von 0 bis 10 bedeuten.

Einige Eigenschaften dieser Verbindungsklasse, etwa deren Verwendung als Katalysatormaterial, als Ionenaustauscher und einige medizinische Anwendungen wurden von W. T. Reichle zusammenfassend beschrieben [**CHEMTECH, Jan. 1986, 58**]. Verschiedene Möglichkeiten zur technischen Herstellung dieser Verbindungen werden in der **DE-OS 20 61 156** angegeben.

Ein gut charakterisierter Vertreter dieser Stoffgruppe ist der als Mineral in der Natur vorkommende Hydrotalcit. Es sind auch synthetisch hergestellte Hydrotalcite bekannt, die z.B. in den **DE-C-15 92 126**, **DE 33 46 943 A1**, **DE 33 06 822 A1** und **EP 0 207 811 A1** beschrieben sind. Hydrotalcit ist ein natürliches Mineral mit der Idealformel

**[Mg₆Al₂(OH)₁₆]CO₃ * 4 H₂O,**

dessen Struktur von derjenigen des Brucits (Mg(OH)₂) abgeleitet ist. Brucit kristallisiert in einer Schichtstruktur mit den Metallionen in Oktaederlücken zwischen zwei Schichten aus dichtgepackten Hydroxylionen, wobei nur jede zweite Schicht der Oktaederlücken besetzt ist. Im Hydrotalcit sind einige Magnesiumionen durch Aluminiumionen ersetzt, wodurch das Schichtpaket eine positive Ladung erhält. Diese wird durch die Anionen ausgeglichen, die sich zusammen mit zeolithischen Kristallwasser in den Zwischenschichten befinden.

Als weitere typische Vertreter dieser Stoffgruppe seien
- Magaldrat [Mg₁₀Al₅(OH)₃₁](SO₄)₂ * n H₂O,
- Pyroaurit [Mg₆Fe₂(OH)₁₆]CO₃ * 4,5 H₂O und
- Hydrocalumit [Ca₂Al(OH)₆]NO₃ * n H₂O
genannt.

Calcinierte Hydrotalcite sind bereits als Ethoxylierungs- und Propoxylierungskatalysatoren mit ausgezeichneten Ergebnissen eingesetzt worden [**DE 38 43 713 A1**]. Sie weisen allerdings den Nachteil auf, daß sie aus den natürlichen und synthetischen Hydrotalciten durch mehrstündiges Erhitzen auf Temperaturen von z.B. 400 bis 600°C, in eine für katalytische Zwecke geeignete, calcinierte Form überführt werden müssen. Weiterhin sind calcinierte Verbindungen anfällig gegen Wasserspuren und das Kohlendioxid aus der Luft (Rückreaktion der Calcinierung), so daß ihr Anwendungsbereich und ihre Lagerstabilität infolge des Aktivitätsverlustes begrenzt sind.

Hydrophobierte Hydrotalcite, die durch die Behandlung von Hydrotalcit mit Anionen von Säuren, z.B. Fettsäuren, entstehen, werden gemäß den [**DE 30 19 632 A1**, **US 4.761.188**, **EP 0 142 773 A1**, **EP 0 189 899 A1**, **EP 0 256 872 A1**] bereits als Stabilisatoren für thermoplastische Harze eingesetzt. Bei dieser Oberflächenbehandlung wird mit anionischen, oberflächenaktiven Mitteln in einer Menge von 1 bis 10 Gew.-% - bezogen auf den Hydrotalcit - gearbeitet.

Aus der Lehre der **DE 37 31 919 A1** ist weiterhin bekannt, daß man Verbindungen der allgemeinen Formel AlₓMg_{y}(OH)_{35-z}R_{z} * n H₂O, worin R das Anion einer Monocarbonsäure bedeutet, als Verdickungs-, Thixotropierungs-, Stabilisierungs- oder Antiabsetzmittel einsetzen kann. Die in dieser Offenlegungsschrift offenbarten Verbindungen werden durch einen vollständigen Austausch von Sulfationen gegen Monocarbonsäureanionen mittels einer wäßrigen Suspension des Alkalisalzes der Monocarbonsäure hergestellt.

Gemäß der **DE 40 10 606 A1** werden hydrophobierte Hydrotalcit-Verbindungen der allgemeinen Formel MgₓAl(OH)_{y}(CO₃)ₘ(A)ₙ * z H₂O, in der A für ein Dianion einer aliphatischen Dicarbonsäure oder für zwei Monoanionen einer aliphatischen Monocarbonsäure stehen, als Alkoxylierungskatalysatoren beschrieben.

Die Aufgabe der vorliegenden Erfindung bestand nunmehr darin, neue hydrophobierte Doppelschichthydroxid-Verbindungen zur Verfügung zu stellen, in denen das dreiwertige Aluminium bzw. zweiwertige Magnesium gegen gleichwertige Metallkationen ersetzt wird und das Verhältnis des auszutauschenden Anions B zum hydrophobierend wirkenden Anion A variiert werden kann.

Weiterhin sollten bekannte Doppelschichthydroxid-Verbindungen, die bisher weder in reiner Form noch durch Calcinierung als Katalysator für Alkoxylierungsreaktionen in Frage kamen, durch Hydrophobierung in eine aktive Katalysatorform überführt werden.

### Beschreibung der Erfindung

Gegenstand der vorliegenden Erfindung sind dementsprechend hydrophobierte Doppelschichthydroxid-Verbindungen der allgemeinen Formel **(I)**,

**[M(II)**_{**1-x**}**M(III)**_{**x**}**(OH)₂]A**_{**a**} **B**_{**b**} *** z H₂O (I)**

in der M(II) ein zweiwertiges Metallkation, ausgewählt aus der von Magnesium, Zink, Calcium, Eisen, Cobalt, Kupfer, Cadmium, Nickel und Mangan gebildeten Gruppe, bedeutet,
in der M(III) ein dreiwertiges Metallkation, ausgewählt aus der von Aluminium, Eisen, Chrom, Mangan, Wismut und Cer gebildeten Gruppe, bedeutet,
in der A für ein Äquivalent eines Monoanions einer aliphatischen Monocarbonsäure mit 2 bis 34 C-Atomen oder für ein Äquivalent eines Dianions einer aliphatischen Dicarbonsäure mit 4 bis 44 C-Atomen steht,
in der B ein Anion, aus der von Carbonat, Hydrogencarbonat, Sulfat, Nitrat, Nitrit, Phosphat, Hydroxid und Halogeniden gebildeten Gruppe, bedeutet
und in der die Bedingungen
0,1 ≦ x ≦ 0,5
0 < a ≦ 0,5
0 ≦ b ≦ 0,5
0 < a + b ≦ 0,5
O ≦ z ≦ 10
gelten, wobei das Zahlenverhältnis von a zu b im Bereich von 0,49 : 0,01 bis 0,05 : 0,45 liegt und Verbindungen, die die Kombinationen von Magnesium und Aluminium mit Carbonat und/oder Sulfat enthalten, ausgeschlossen sind.

Ein weiterer Gegenstand der vorliegenden Erfindung ist ein Verfahren zur Herstellung von hydrophobierten Doppelschichthydroxid-Verbindungen der allgemeinen Formel **(I)**, das sich dadurch auszeichnet, daß man Doppelschichthydroxid-Verbindungen der allgemeinen Formel **(II)**,

**[M(II)**_{**1-x**} **M(III)**_{**x**}**(OH)₂] B * z H₂O (II)**

in der M(II), M(III), B, x und z die oben genannte Bedeutung besitzen, ausschließlich der Verbindungen, die die Kombination von Magnesium und Aluminium mit Carbonat und/oder Sulfat enthalten, mit wenigstens einer aliphatischen Monocarbonsäure mit 2 bis 34 C-Atomen und/oder wenigstens einer aliphatischen Dicarbonsäure mit 4 bis 44 C-Atomen entweder
a) in einem organischen Lösungsmittel umsetzt und das Lösungsmittel durch Trocknen bei 20 bis 150 °C entfernt oder
b) durch Rühren oder Kneten direkt miteinander umsetzt oder
c) die Doppelschichthydroxid-Verbindungen der allgemeinen Formel **(II)** mit einem Alkali- und/oder Erdalkalimetallsalz der Mono- und/oder Dicarbonsäuren in wäßriger Suspension umsetzt.

Die Anionen A der Monocarbonsäuren, die für die Hydrophobierung von Doppelschichthydroxid-Verbindungen eingesetzt werden können, sind z.B. diejenigen der Monocarbonsäuren mit 2 bis 34 C-Atomen, vorzugsweise Fettsäuren mit 6 bis 22 Kohlenstoffatomen natürlicher oder synthetischer Herkunft, insbesondere geradkettige, gesättigte oder ungesättigte Fettsäuren einschließlich technischer Gemische derselben, wie sie durch Fettspaltung aus tierischen und/oder pflanzlichen Fetten und Ölen zugänglich sind, z.B. aus Kokosöl, Palmkernöl, Palmöl, Soyaöl, Sonnenblumenöl, Rüböl, Baumwollsaatöl, Fischöl, Rindertalg und Schweineschmalz.

Typische Beispiele sind Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Arachinsäure, Gadoleinsäure, Behensäure und Erucasäure; weiterhin methylverzweigte, gesättigte und ungesättigte Fettsäuren mit 10 bis 22 Kohlenstoffatomen, die bei der Dimerisierung von den entsprechenden ungesättigten Fettsäuren als Nebenprodukte entstehen. Es können aber auch Monocarbonsäuren mit 2 bis 5 Kohlenstoffatomen, wie vorzugsweise Essigsäure oder Propionsäure eingesetzt werden. Besonders bevorzugt werden allerdings Laurinsäure und Stearinsäure eingesetzt. Typische Beispiele für Dicarbonsäuren A, die für die Hydrophobierung von Doppelschichthydroxid-Verbindungen geeignet sind, sind Bernsteinsäure, Maleinsäure, Fumarsäure, Adipinsäure, Pimelinsäure, Suberinsäure (Korksäure), Sebacinsäure und dergleichen; weiterhin auch sogenannte Dimerfettsäuren, die z.B. aus Öl- oder Tallölfettsäuren erhalten werden können und bis zu 44 Kohlenstoffatome aufweisen. Weiterhin können die Dimerisierungsprodukte von gesättigten, einfach und/oder mehrfach ungesättigten C₁₆-C₂₂-Monomerfettsäuren, wie Palmitoleinsäure, Ölsäure, Gadoleinsäure, Erucasäure, Ricinolsäure, Linolsäure, Linolensäure, Arachidonsäure und Behensäure eingesetzt werden.

In einer bevorzugten Ausführungsform der vorliegenden Erfindung werden zur Hydrophobierung der Doppelschichthydroxid-Verbindungen die Anionen A von Monocarbonsäuren mit 6 bis 22 Kohlenstoffatomen oder von Dicarbonsäuren einschließlich Dimerfettsäuren, mit 8 bis 36 Kohlenstoffatomen eingesetzt.

Im Sinne der vorliegenden Erfindung werden die Anionen B, je nach der Herkunft der Doppelschichthydroxid-Verbindungen der allgemeinen Formel II, aus der von Carbonat, Hydrogencarbonat, Sulfat, Nitrat, Nitrit, Phosphat, Hydroxid und Halogeniden gebildeten Gruppe ausgewählt.

Im Rahmen der vorliegenden Erfindung soll in den hydrophobierten Doppelschichthydroxid-Verbindungen der allgemeinen Formel **(I)** das Zahlenverhältnis von a zu b vorzugsweise im Bereich von 0,3 : 0,02 bis 0,05 : 0,25, liegen. Erfindungsgemäß wird ein hoher Austausch- bzw. Hydrophobierungsgrad in den Verbindungen der allgemeinen Formel **(I)** angestrebt, so daß möglichst alle ein- oder mehrbasischen, anorganischen Anionen B in den Verbindungen der allgemeinen Formel **(I)** ausgetauscht werden. Eine vollständige Hydrophobierung tritt erfindungsgemäß insbesondere in den Fällen ein, in denen a > x erreicht wird.

Deswegen sind auch hydrophobierte Doppelschichthydroxid-Verbindungen der allgemeinen Formel **(I)** bevorzugt, die, bezogen auf ihr Gesamtgewicht, 15 - 70 Gew.-%, insbesondere 20 bis 50 Gew.-% der Anionen A von Monocarbonsäuren mit 6 bis 22 Kohlenstoffatomen oder 10 bis 60 Gew.-%, insbesondere 15 bis 50 Gew.-% der Dianionen A der Dicarbonsäuren mit 8 bis 36 Kohlenstoffatomen enthalten.

Die Herstellung der hydrophobierten Doppelschichthydroxid-Verbindungen kann auf verschiedenen Wegen erfolgen, z.B. durch direkte Umsetzung von natürlichen oder synthetischen Doppelschichthydroxid-Verbindungen mit Mono- und/oder Dicarbonsäuren im Kneter oder in Anwesenheit von organischen Lösungsmitteln sowie durch Umsetzung der DoppelschichthydroxidVerbindungen mit einer wäßrigen Suspension eines Mono- und/oder Dicarbonsäuresalzes.

In einer bevorzugten Ausführungsform der vorliegenden Erfindung werden die hydrophobierten Doppelschichthydroxid- Verbindungen in einem niedrigsiedenden organischen Lösungsmittel, vorzugsweise Alkohole mit 1 bis 6 C-Atomen, offenkettige und cyclische Ether und/oder Ketone, durch Umsetzung von Monocarbonsäuren und/oder Dicarbonsäuren mit den DoppelschichthydroxidVerbindungen der allgemeinen Formel **(I)** hergestellt. Besonders bevorzugt wird die Hydrophobierung in iso-Propanol, Diethylether, Tetrahydrofuran und/oder Aceton durchgeführt.

In einer weiteren bevorzugten Ausführungsform wird mit einem Molverhältnis zwischen der eingesetzten Doppelschichthydroxid-Verbindung und der Monocarbonsäure (bzw. Dicarbonsäure) von 6 : 1 (3 : 1) bis 1 : 10 (1 : 5), vorzugsweise 3 : 1 (1,5 : 1) bis 1 : 3 (1 : 1,5) gearbeitet. Wie dem Fachmann bekannt ist, wird man das Molverhältnis auf die beim Austausch zur Verfügung stehenden Anionen der jeweilig eingesetzten Doppelschichthydroxid-Verbindungen einstellen müssen.

Das erfindungsgemäße Verfahren kann bei Temperaturen von 20 bis 120 °C, vorzugsweise bei 40 bis 100 °C durchgeführt werden, wobei man im allgemeinen die Doppelschichthydroxid-Verbindung und die Carbonsäure (bzw. Dicarbonsäure) für 0,5 bis 8 Stunden, vorzugsweise 1 bis 6 Stunden, im jeweiligen organischen Lösungsmittel unter Rückfluß erhitzt.

In einer weiteren bevorzugten Ausführungsform der vorliegenden Erfindung wird das organische Lösungsmittel in 0,5 bis 3 Stunden, vorzugsweise 1 bis 2 Stunden, bei Temperaturen von 20 bis 150 °C, vorzugsweise 50 bis 120 °C, entfernt.

Die erfindungsgemäßen, hydrophobierten Doppelschichthydroxid-Verbindungen können weiterhin auch durch direkte Umsetzung von Doppelschichthydroxid-Verbindungen mit Mono- und/oder Dicarbonsäuren ohne Zusatz eines Lösungsmittels mit einem beliebigen Rührwerkzeug, vorzugsweise einem Kneter, erhalten werden.

Prinzipiell lassen sich die erfindungsgemäßen Verbindungen der allgemeinen Formel **(I)** auch analog dem aus der Lehre der **DE 37 31 919 A1** bekannten Verfahren durch Umsetzung von Doppelschichthydroxid-Verbindungen mit einer wäßrigen Suspension eines Alkali- und/oder Erdalkalimetallsalzes, vorzugsweise Natriumsalze einer Mono- und/oder Dicarbonsäure, herstellen.

Schließlich können die hydrophobierten Doppelschichthydroxid-Verbindungen auch aus calzinierten Doppelschichthydroxid-Verbindungen durch Umsetzung derselben mit den Mono- oder Dicarbonsäuren erhalten werden; dabei können unter Luft- bzw. CO₂-Ausschluß oder in Gegenwart von Kohlendioxid carbonatfreie oder carbonathaltige Produkte erhalten werden.

Anhand von Röntgenbeugungsdiagrammen konnte gezeigt werden, daß in den hydrophobierten Doppelschichthydroxid-Verbindungen die Schichtstruktur unter Aufweitung der Schichtabstände erhalten geblieben ist.

Der stöchiometrische Wassergehalt der hydrophobierten Doppelschichthydroxid-Verbindungen kann - in Abhängigkeit von der Art der Herstellung und den Trocknungsbedingungen - im Bereich von 0 bis 10 Moleküle liegen; bevorzugt ist ein Bereich von 0 bis 4 Molekülen, der sich im allgemeinen einstellt, wenn man die hydrophobierten Doppelschichthydroxid-Verbindungen bei Temperaturen im Bereich 100 bis 250°C, vorzugsweise von 150 bis 220°C bis zur Gewichtskonstanz trocknet, so daß eine besonders hohe katalytische Aktivität gewährleistet werden kann.

Ein weiterer Gegenstand der vorliegenden Erfindung betrifft die Verwendung von hydrophobierten Doppelschichthydroxid-Verbindungen der allgemeinen Formel **(I)**, ausschließlich der Verbindungen, die die Kombination von Magnesium und Aluminium mit Carbonat enthalten, als Alkoxylierungskatalysator für Verbindungen mit aktiven H-Atomen oder für Fettsäureester.

Die vorliegende Erfindung beruht dabei auf der Erkenntnis, daß sich hydrophobierte Doppelschichthydroxid-Verbindungen für die Ethoxylierung bzw. Propoxylierung von Verbindungen mit aktiven H-Atomen sowie von Fettsäureestern eignen; diese Erkenntnis ist überraschend, denn unbehandelte natürliche bzw. synthetische Doppelschichthydroxid-Verbindungen, d.h. solche in nicht-calcinierter Form, als auch eine Vielzahl von calcinierten Verbindungen sind als Ethoxylierungs- bzw. Propoxylierungskatalysatoren nicht aktiv.

Unter Verbindungen mit aktiven H-Atomen im Sinne der Erfindung sind z.B. Fettalkohole, Fettsäuren und Amine zu verstehen, die bei der Ethoxylierung bzw. Propoxylierung nichtionische Detergentien bilden. Ein typisches Beispiel hierfür ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u.a. die folgenden eingesetzt worden:
- Calcium- und Strontiumhydroxide, -alkoxide und -phenoxide [**EP 0 092 256 A1**],
- Calciumalkoxide [**EP 0 091 146 A1**],
- Bariumhydroxid [**EP 0 115 083 B1**],
- basische Magnesiumverbindungen, z.B. Alkoxide [**EP 0 082 569 A1**],
- Magnesium- und Calciumfettsäuresalze [**EP 0 085 167 A1**].

Die vorgenannten Katalysatoren weisen u.a. den Nachteil auf, daß sie schlecht in das Reaktionssystem einarbeitbar und/oder schwierig herstellbar sind. Gebräuchliche Alkoxylierungskatalysatoren sind weiterhin Kaliumhydroxid und Natriummethylat.

Für Fettalkoholpolyalkoxylate ist eine enge Bandbreite des Polyalkoxylierungsgrades von besonderer Bedeutung [**JAOCS, 63, 691 (1986), HAPPI, 52 (1986)**. Die sogenannten "narrow-range" -Alkoxylate weisen demnach insbesondere die folgenden Vorteile auf:
- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Unter erfindungsgemäßer Verwendung hydrophobierter Doppelschichthydroxid-Verbindungen als Katalysatoren können Verbindungen mit aktiven H-Atomen und Fettsäureester bei kurzen Reaktionszeiten mit hohen Ausbeuten polyalkoxyliert werden; dabei weisen die Reaktionsprodukte eine enge Bandbreite bzw. Homologenverteilung auf, wobei die Verteilungskurve der nach Poisson berechneten sehr nahe kommt. Die erfindungsgemäß eingesetzten hydrophobierten Doppelschichthydroxid-Verbindungen weisen den Vorteil auf, daß sie in das Reaktionsgemisch der Alkoxylierung leicht eingearbeitet werden und wegen ihrer Unlöslichkeit in dem Reaktionsgemisch durch einfache Maßnahmen wieder abgetrennt werden können. Sie können jedoch auch in dem Reaktionsgemisch verbleiben, wenn ihre Anwesenheit bei der Weiterverwendung der Reaktionsprodukte nicht stört.

Beispiele für erfindungsgemäß unter Verwendung von hydrophobierten Doppelschichtverbindungen alkoxylierbare Verbindungen sind im folgenden aufgeführt.

**Fettsäuren** mit 6 bis 22 Kohlenstoffatomen natürlicher oder synthetischer Herkunft, insbesondere geradkettige, gesättigte oder ungesättigte Fettsäuren einschließlich technischer Gemische derselben, wie sie durch Fettspaltung aus tierischen und/oder pflanzlichen Fetten und Ölen zugänglich sind, z.B. aus Kokosöl, Palmkernöl, Palmöl, Soyaöl, Sonnenblumenöl, Rüböl, Baumwollsaatöl, Fischöl, Rindertalg und Schweineschmalz.

Typische Beispiele sind Capronsäure, Caprylsäure, Caprinsäure, Laurinsäure, Myrisitinsäure, Palmitinsäure, Palmitoleinsäure, Stearinsäure, Ölsäure, elaidinsäure, Petroselinsäure, Linolsäure, Linolensäure, Arachinsäure, Gadoleinsäure, Behensäure, Erucasäure, Arachidonsäure und Clupanodonsäure; weiterhin methylverzweigte, gesättigte und ungesättigte Fettsäuren mit 10 bis 22 Kohlenstoffatomen, die bei der Dimerisierung von den entsprechenden ungesättigten Fettsäuren als Nebenprodukte entstehen, und Monocarbonsäuren mit 1 bis 7 Kohlenstoffatomen.

**Hydroxyfettsäuren** natürlicher oder synthetischer Herkunft, insbesondere mit 16 bis 22 Kohlenstoffatomen, z.B. Ricinolsäure oder 12-Hydroxystearinsäure.

**Fettsäureamide**. Derivate der oben genannten geradkettigen, gesättigten oder ungesättigten Fettsäuren mit Ammoniak oder primären aliphatischen Aminen mit 1 bis 4 Kohlenstoffatomen in dem aliphatischen Substituenten.

**Alkanole**. Gesättigte oder ungesättigte Monoalkanole, insbesondere Hydrierungsprodukte der oben genannten geradkettigen, gesättigten oder ungesättigten Fettsäuren bzw. Derivate derselben wie Methylester oder Glyceride; aliphatische oder cyclische Alkanole mit 2 bis 6 Kohlenstoffatomen, z.B. Ethanol, Propanol, Butanol, Hexanol und Cyclohexanol; einschließlich der von den vorgenannten Monoalkanolen abgeleiteten Guerbet-Alkohole.

**Alkylphenole**. Mono-, Di- oder Trialkylphenole, insbesondere mit 4 bis 12 Kohlenstoffatomen in den Alkylgruppen.

**Polyglykole**. Polyethylen- oder Polypropylenglykole (durchschnittlicher Polymerisationsgrad 2 bis 2000).

**Fettamine**. Insbesondere primäre Fettamine, die aus Nitrilen der oben genannten geradkettigen, gesättigten oder ungesättigten Fettsäuren oder den entsprechenden Fettalkoholen zugänglich sind; weiterhin auch Mono- und Dialkylamine mit C₁-C₆-Alkylgruppen.

**Fettsäurealkanolamide**. Derivate der oben genannten geradkettigen, gesättigten oder ungesättigten Fettsäuren mit Mono- oder Dialkanolaminen, insbesondere Mono- oder Diethanolamin.
**vicinale hydroxy- bzw. alkoxy-substituierte Alkane**. Ringöffnungsprodukte von 1,2-Epoxyalkangemischen mit 12 bis 22 Kohlenstoffatomen in der Kette mit mehrwertigen Alkanolen mit 2 bis 12 Kohlenstoffatomen und 2 bis 6 Hydroxylgruppen; diese Verbindungen jedoch nur, wenn sie mit Ethylenoxid oder zunächst mit Ethylenoxid und anschließend mit Propylenoxid umgesetzt werden.

**Fettsäureester**, gebildet von den gegebenenfalls methylverzweigten Fettsäuren bzw. Monocarbonsäuren und Hydroxyfettsäuren gemäß der obigen Aufstellung und den Alkanolen der obigen Aufstellung; weiterhin Ester dieser Säuren mit Polyolen, z.B. mit Ethylenglykol, 1,2-Propylenglykol, 1,2-Butylenglykol, Neopentylglykol, Glycerin, Diglycerin, Triglycerin, Tetraglycerin, Trimethylolpropan, Di-trimethylolpropan, Pentaerythrit, Di-Pentaerythrit, und Zuckeralkohole, insbesondere Sorbitan.

Wie bereits eingangs ausgeführt wurde, können im Falle von Estern der obengenannten Fettsäuren mit den vorgenannten Polyolen diese auch als Partialester bzw.

Partialester-enthaltende technische Estergemische, insbesondere in Form von Glyceriden, vorliegen.

Bevorzugte Fettsäureester für die erfindungsgemäße Ethoxylierung und/oder Propoxylierung sind von gesättigten oder ungesättigten, gegebenenfalls methylverzweigten oder gegebenenfalls hydroxysubstituierten Fettsäuren mit 8 bis 22 Kohlenstoffatomen mit Alkanolen mit 1 bis 4 Kohlenstoffatomen oder mit Glycerin gebildet.

Die Struktur der erfindungsgemäß erhaltenen ethoxylierten bzw. propoxylierten Fettsäureester ist nicht immer eindeutig feststellbar. Während Ester aus Fettsäuren und Monoalkanolen bzw. Vollester derselben mit Polyolen unter Einschub von Ethylenoxy- und/oder Propylenoxy-Einheiten in die Esterbindung reagieren dürften, läßt sich nicht feststellen, zu welchen Reaktionsprodukten die Reaktion von Ethylenoxid und/oder Propylenoxid mit Partialestern von Fettsäuren und Polyolen oder von hydroxy-substituierten Fettsäuren und Monoalkanolen führt; hier sind auch Reaktionen an den freien OH-Gruppen denkbar, und zwar insbesondere bei freien, primären OH-Gruppen.

Die erfindungsgemäß unter Verwendung von hydrophobierten Doppelschichthydroxid-Verbindungen herzustellenden Derivate sind handelsübliche Produkte, so daß sich eine nähere Erläuterung erübrigt. Sie werden durchweg durch Ethoxylierung und/oder Propoxylierung aktive Wasserstoffatome aufweisender Ausgangsverbindungen bzw. von Fettsäureestern hergestellt. Typische Vertreter sind beispielsweise ein Anlagerungsprodukt von 9 Mol Ethylenoxid an Kokosölfettsäure, ein Anlagerungsprodukt von 2 Mol Ethylenoxid an ein Fettalkoholgemisch der Kettenlänge C12-14, ein Anlagerungsprodukt von 3 Mol Ethylenoxid und 8 Mol Propylenoxid an ein Fettalkoholgemisch der Kettenlänge C12-18, ein Anlagerungsprodukt von 10 Mol Ethylenoxid an Nonylphenol, ein Anlagerungsprodukt von 7,3 Mol Ethylenoxid an Glycerin, ein Anlagerungsprodukt von 10 Mol Ethylenoxid an ein Diolgemisch, das durch Umsetzung eines 1,2-Epoxyalkangemisches der Kettenlänge C12-16 mit Ethylenglykol erhalten wurde, ein Anlagerungsprodukt von 12 Mol Ethylenoxid an ein Fettamingemisch der Kettenlänge C10-18 und ein Anlagerungsprodukt von 4 Mol Ethylenoxid an Kokosfettsäuremonoethanolamid; weiterhin Anlagerungsprodukte von 41 mol Ethylenoxid an Ricinusöl, Anlagerungsprodukte von 25 mol Ethylenoxid an gehärtetes Ricinusöl, Anlagerungsprodukte von 7 Gew.-Teilen Ethylenoxid an 10 Gew.-Teile eines Palmitinsäure-/Stearinsäuremono-/diglyceridgemisches mit einem Anteil von 40 bis 45 Gew.-% Monoglycerid und Anlagerungsprodukte von 20 mol Ethylenoxid an Sorbitanmonostearat.

Gemäß einer bevorzugten Ausführungsform der Erfindung sind die unter Verwendung der hydrophobierten DoppelschichthydroxidVerbindungen ethoxylierbaren bzw. propoxylierbaren Verbindungen mit aktiven H-Atomen aus der von Fettsäuren, Hydroxyfettsäuren, Fettsäureamiden, Alkanolen, Alkylphenolen, Polyglykolen, Fettaminen, Fettsäurealkanolamiden oder vicinal hydroxy- bzw. alkoxy-substituierten Alkanen gebildeten Gruppe ausgewählt.

Weiterhin bevorzugt ist, die hydrophobierten Doppelschichthydroxid-Verbindungen in einer Menge von 0,1 bis 3, vorzugsweise 0,5 bis 2 Gew.-%, bezogen auf das Endprodukt der Ethoxylierung bzw. Propoxylierung, einzusetzen.

In einer bevorzugten Ausführungsform der Erfindung wird die Reaktion zwischen Verbindungen mit aktiven Wasserstoffatomen oder Fettsäureestern einerseits und Ethylen- und/ oder Propylenoxid andererseits in Gegenwart der erfindungsgemäßen hydrophobierten Doppelschichhydroxidverbindungen bei einer Temperatur von 125 bis 180, vorzugsweise 150 bis 160°C und einem Druck von 1 bis 5 bar durchgeführt. Die dafür benötigte Zeitspanne ergibt sich aus dem gewünschten Alkoxylierungsgrad und beträgt üblicherweise 0,5 bis 5 Stunden.

Die folgenden Beispiele sollen den Gegenstand der Erfindung näher erläutern, ohne ihn darauf einzuschränken.

### Beispiele

### Beispiel 1:

**Umsetzung von Magaldrat mit Natriumlaurat**. 20 g Magaldrat (kommerzielles Produkt der Fa.Giulini, Ludwigshafen) der Idealformel [Mg₁₀Al₅(OH)₃₁](SO₄)₂ * z H₂O wurden in 200 ml Wasser suspendiert und mit einer Lösung von 7,6 g Natriumlaurat in 70 ml Wasser versetzt und für 15 Stunden bei 70 °C gerührt. Nach dem Abfiltrieren und Auswaschen wurde der weiße Niederschlag bei 200 °C und 100 mbar getrocknet.

| | |
|---|---|
| Auswaage | 20,2 g hydrophobierter Magaldrat |
| Analytik | 17,7 Gew.-% Mg |
| | 9,5 Gew.-% Al |
| | 18,7 Gew.-% C |
| | 1,8 Gew.-% SO₄ |
| Mg/Al-Verhältnis | 2,1 |
| Al/Laurat-Verhältnis | 2,71 |
| 25,9 Gew.-% Laurat, x = 0,33; a = 0,14; b = 0,04 | |

### Beispiel 2:

**Umsetzung von chloridhaltigem Hydrotalcit mit Natriumlaurat**. 20 g chloridhaltigen Hydrotalcit [Mg₆Al₂(OH)₁₆]Cl₂ * 4 H₂O wurden in 200 ml Wasser suspendiert und mit einer Lösung aus 15 g Natriumlaurat in 100 ml Wasser versetzt und für 3 Stunden bei 70 °C gerührt. Nach dem Abfiltrieren und Auswaschen wurde der Niederschlag bei 105 °C und 100 mbar getrocknet. Als Katalysator wurde das Produkt bei 200 °C und 100 mbar noch nachgetrocknet.

| | |
|---|---|
| Auswaage | 26,3 g hydrophobierter Hydrotalcit |
| Analytik | 12,7 Gew.-% Mg |
| | 7,6 Gew.-% Al |
| | 25,7 Gew.-% C |
| | 0,5 Gew.-% Cl |
| Mg/Al-Verhältnis | 1,9 |
| Al/Laurat-Verhältnis | 1,58 |
| 35,5 Gew.-% Laura, x = 0,35; a = 0,22; b = 0,02 | |

### Beispiel 3:

**Umsetzung von nitrathaltigem Hydrotalcit mit Natriumlaurat**. 20 g eines getrockneten, nitrathaltigen Hydrotalcits der Idealformel [Mg₆Al₂(OH)₁₆](NO₃)₂ * n H₂O, hergestellt durch Fällung einer Magnesium- und Aluminiumnitrat - Lösung mit überschüssigem Ammoniak, wurden in 200 ml Wasser suspendiert und mit einer Lösung aus 13,4 g Natriumlaurat in 100 ml Wasser versetzt und über Nacht bei 70 °C gerührt. Nach dem Abfiltrieren und Auswaschen wurde der Niederschlag bei 105 °C und 100 mbar getrocknet.

| | |
|---|---|
| Auswaage | 27,4 g hydrophobierter Hydrotalcit |
| Analysen | 17,3 Gew-% Mg |
| | 6,9 Gew.-% Al |
| | 27,1 Gew.-% C |
| | 6,8 Gew.-% H |
| | 3,0 Gew.-% NO₃⁻ |
| Mg/Al-Verhältnis | 2,78 |
| Al/Laurat-Verhältnis | 1,36 |
| 37,5 Gew.-% Laurat, x = 0,26; a = 0,19; b = 0,05 | |

### Beispiel 4:

**Umsetzung von Pyroaurit mit Laurinsäure**. 10 g eines syntheischen Pyroaurits der Idealformel [Mg₆Fe₂(OH)₁₆]CO₃ * 4,5 H₂O wurden in 150 ml Isopropanol suspendiert und mit 6,05 g Laurinsäure in 50 ml Isopropanol bei Raumtemperatur versetzt. Es wurde auf Rückflußtemperatur erhitzt und für 5 h bei dieser Temperatur gehalten. Nach dem Abfiltrieren und Waschen mit Isopropanol wurde das Produkt bei 105 °C und 100 mbar getrocknet.

| | |
|---|---|
| Auswaage | 13,5 g hydrophobierter Pyroaurit |
| Analysen | 16,8 Gew.-% Mg |
| | 12,7 Gew.-% Fe |
| | 34,9 Gew.-% C |
| | 6,8 Gew.-% H |
| | <0,1 Gew.-% CO₃²⁻ |
| Mg/Fe-Verhältnis | 3,04 |
| Fe/Laurat-Verhältnis | 0,94 |
| 48,3 Gew.-% Laurat, x = 0,25; a = 0,26 | |

### Beispiel 5:

**Umsetzung der Zn/Al-Phase mit Laurinsäure**. Die Zn/Al-Phase der Idealformel [Zn₁₋ₓAlₓ(OH)₂](CO₃)_{x/2} * n H₂O mit x = 0,17 wurde synthetisiert durch Reaktion von Zinkoxid mit wäßriger, natriumcarbonathaltiger Aluminiumnitrat-Lösung. Nach Filtration und Waschen wurde das Produkt getrocknet. 40 g dieser Phase wurden mit 18,8 g Laurinsäure in 300 ml Isopropanol unter Rückflußtemperatur umgesetzt. Nach dem Abfiltrieren und Waschen mit Isopropanol wurde das Produkt bei 105 °C und 100 mbar getrocknet.

| | |
|---|---|
| Auswaage | 51,9 g hydrophobierte Zn/Al-Phase |
| Analysen | 39,1 Gew.-% Zn |
| | 3,3 Gew.-% Al |
| | 30,4 Gew.-% C |
| | 5,8 Gew.-% H |
| | <0,1 Gew.-% CO₃²⁻ |
| Zn/Al-Verhältnis | 4,89; |
| Al/Laurat-Verhältnis | 0,58; |
| 42 Gew.-% Laurat, x = 0,17; a = 0,29 | |

### Beispiel 6:

**Umsetzung von Hydrocalumit mit Laurinsäure**. Hydrocalumit der Idealformel [Ca₂Al(OH)₆]NO₃ * n H₂O wurde durch die Reaktion von Calcium- und Aluminiumnitrat in alkalischer Lösung unter Luftausschluß synthetisiert. Nach Filtration und Waschen wurden 20 g des getrockneten Produktes mit 12,2 g Laurinsäure in 300 ml Wasser für 5 h bei 70 °C umgesetzt, gewaschen und bei 110 °C und 100 mbar getrocknet.

| | |
|---|---|
| Auswaage | 27,3 g hydrophobierter Hydrocalumit |
| Analysen | 30,5 Gew.-% Ca |
| | 9,7 Gew.-% Al |
| | 34,5 Gew.-% C |
| | 7,2 Gew.-% H |
| Ca/Al-Verhältnis | 2,12; |
| Al/Laurat-Verhältnis | 1,50 |
| 47,7 Gew.-% Laurat, x = 0,32; a = 0,21 | |

### Beispiel 7:

**Herstellung einer Zn/Al-Phase und Umsetzung mit Laurinsäure**. Eine Lösung aus 178,5 g Zinknitrat (0,6 mol) und 75,0 g Aluminiumnitrat (0,2 mol) wurde zu einer alkalischen Natriumcarbonatlösung gegeben, sodaß der pH-Wert immer größer 9 war. Nach vollendeter Zugabe wurde für 5 Stunden auf 70 °C erhitzt, danach abfiltriert und gewaschen. Bis zur Massenkonstanz wurde das farblose Pordukt bei 110 °C getrocknet.

| | |
|---|---|
| Auswaage: | 70 g farbloses Pulver |
| Analytik: | 33,5 Gew.-% Zn |
| | 16,5 Gew.-% Al (x = 0,5) |

10 g des Pulvers wurden in 200 ml Tetrahydrofuran mit 1,25 g Laurinsäure unter Rückfluß für 5 h bei 70 °C umgesetzt, danach abfiltriert, gewaschen und bei 110 °C und 100 mbar getrocknet.

| | |
|---|---|
| Auswaage: | 10,9 g teilhydrophobierte Zn/Al-Phase |
| Analytik | 30,5 Gew.-% Zn |
| | 15,3 Gew.-% Al |
| | 7,0 Gew.-% C |
| | 8,0 Gew.-% CO₃²⁻ |
| Al/Laurat-Verhältnis | 11,76 |
| 9,61 Gew.-% Laurat, x = 0,5; a = 0,05; b = 0,24 | |

### Beispiel 8:

Beispiel 3 wurde unter Verwendung 8,2 g Natriumcapronat wiederholt.

| | |
|---|---|
| Auswaage | 24,6 g hydrophobierter Hydrotalcit |
| Analytik | 22,5 Gew.-% Mg |
| | 7,0 Gew.-% Al |
| | 5,1 Gew.-% C |
| | 8,5 Gew.-% NO₃⁻ |
| Al/Capronat-Verhältnis | 3,66 |
| 8,17 Gew.-% Capronat, x = 0,22; a = 0,08; b = 0,12 | |

### Beispiel 9:

Beispiel 3 wurde unter Verwendung von 18,4 Natriumsteart wiederholt.

| | |
|---|---|
| Auswaage | 35,4 g hydrophobierter Hydrotalcit |
| Analytik | 12,9 Gew.-% Mg |
| | 4,0 Gew.-% Al |
| | 32,6 Gew.-% C |
| | 4,7 Gew.-% NO₃⁻ |
| Al/Stearat-Verhältnis | 0,98 |
| 42,8 Gew.-% Stearat, x = 0,22; a = 0,22; b = 0,10 | |

### Beispiel 10:

Beispiel 3 wurde unter Verwendung von 36,1 g des Natriumsalzes einer C₃₆-Dimerfettsäure wiederholt.

| | |
|---|---|
| Auswaage | 30,7 g hydrophobierter Hydrotalcit |
| Analytik | 21,1 Gew.-% Mg |
| | 6,5 Gew.-% Al |
| | 11,1 Gew.-% C |
| | 7,8 Gew.-% NO₃⁻ |
| Al/Dicarboxylat-Verhältnis | 9,38 |
| 14,5 Gew.-% Dicarboxylat, x = 0,22; a = 0,06; b = 0,16 | |

### Beispiel 11:

**Umsetzung einer Zn/Al-Phase mit Stearinsäure im Kneter**. 60 g [Zn₁₋ₓAlₓ(OH)₂](CO₂)_{x/2} * z H₂O mit x = 0,24 wurden mit 20,1 g Stearinsäure bei 80 °C in einem Kneter miteinander für 3 h zur Reaktion gebracht. Dabei resultierte ein farbloses Pulver.

| | |
|---|---|
| Auswaage | 79,0 g hydrophobierte Zn/Al-Phase |
| Analytik | 38,6 Gew.-% Zn |
| | 5,1 Gew.-% Al |
| | 20,7 Gew.-% C |
| Al/Stearat-Verhältnis | 1,97; |
| 27,1 Gew.-% Stearat, x = 0,24; a = 0,13 | |

### Beispiel 12:

**Umsetzung von nitrathaltigem Hydrotalcit mit Stearinsäure im Kneter**. 40 g der Hydrotalcit-Phase [Mg₁₋ₓAlₓ(OH)₂](NO₃)ₓ * z H₂O mit x = 0,25 wurden mit 40 ml Isopropanol zu einer pastenförmigen Konsistenz verknetet und anschließend mit 8,5 g Stearinsäure versetzt. Nach Zugabe von 5 ml konzentriertem Ammoniak (25 %-ig) wurde der Kneter auf 80 °C erwärmt und bis zur Trocknung verknetet.

| | |
|---|---|
| Auswaage | 47,0 g hydrophobierter Hydrotalcit |

Zur Zersetzung des Ammoniumnitrats wurde das Produkt vorsichtig bei 200 °C getrocknet.

| | |
|---|---|
| Analytik | 18,3 Gew.-% Mg |
| | 6,9 Gew.-% Al |
| | 15,5 Gew.-% C |
| | 7,0 Gew.-% NO₃⁻ |
| Al/Stearat-Verhältnis | 3,57 |
| 20,3 Gew.-% Stearat, x = 0,25; a = 0,07; b = 0,11 | |

### Beispiel 13:

**Herstellung einer Mg/Bi-Phase und Umsetzung mit Laurinsäure**. Eine Lösung aus 237 g Wismutnitrat (0,6 mol) in verdünnter Salpetersäure wurden mit einer Lösung von 461,5 g Magnesiumnitrat (1,8 mol) vereinigt und verrührt. Diese Mischung wurde zu einer alkalischen Natriumcarbonat-Lösung, die stets in Überschuß vorhanden war, unter intensivem Rühren gegeben. Dabei trat eine gelbliche Fällung auf, die im Laufe der Reaktionszeit farblos wurde. Zur Reaktionsvervollständigung erwärmte man die Suspension auf 80 °C. Nach dem Abkühlen wurde abfiltriert, gewaschen und getrocknet. Zur Auswaage kamen 285 g eines farblosen Pulvers.

| | |
|---|---|
| Analytik | 10,0 Gew.-% Mg |
| | 56,0 Gew.-% Bi, x = 0,39 |

135 g des Pulvers wurden in 400 ml Isopropanol suspendiert und mit einer Lösung aus 56,1 g Laurinsäure in 200 ml Isopropanol versetzt. Die erhaltene Suspension wurde für 5 h auf Rückfluß erhitzt, anschließend filtriert, gewaschen und getrocknet.

| | |
|---|---|
| Auswaage | 180 g hydrophobierte Mg/Bi-Phase |
| Analytik | 7,4 Gew.-% Mg |
| | 47,2 Gew.-% Bi |
| | 23,2 Gew.-% C |
| Bi/Laurat-Verhältnis | 1,40; |
| 32,1 Gew.-% Laurat, x = 0,43; a = 0,30 | |

**Allgemeine Herstellvorschrift zur Herstellung von Alkoxylaten von Verbindungen mit aktiven H-Atomen unter Verwendung der erfindungsgemäßen Katalysatoren:**
Die zu alkoxylierende Verbindung wurde in einem Druckreaktor mit Rührwerk vorgelegt und mit den in den Beispielen 1 bis 8 hergestellten Katalysatoren, die bei 200 °C und 100 mbar vorgetrocknet wurden, versetzt. Der Reaktor wurde mit Stickstoff gespült und 30 min bei einer Temperatur von 100 °C evakuiert. Anschließend wurde die Temperatur auf ca. 150 - 160 °C erhöht und Ethylenoxid bzw. Propylenoxid bei einem Druck von max. 4 - 5 bar aufgedrückt. Die Reaktionstemperatur der exothermen Reaktion sollte 180 °C nicht überschreiten. Nach Beendigung der Reaktion ließ man 30 min nachreagieren und evakuierte ebenfalls 30 min bei 120 °C. Man erhielt das gewünschte Reaktionsprodukt nach Abkühlen und Abfiltrieren des heterogenen Katalysators.

Unter Verwendung eines handelsüblichen C₁₂/C₁₄-Fettalkoholschnitts (Lorol^{(R)} Spezial, Hydroxylzahl 280, Fa.Henkel KGaA, Ansatzgröße jeweils 300 g Fettalkohol) wurde nach der obigen Vorschrift ein Fettalkoholethoxylat durch Anlagerung von 3 mol Ethylenoxid pro Mol Fettalkohol hergestellt.

In der folgenden Tabelle 1 sind die jeweils eingesetzten Katalysatoren, die Einsatzkonzentration, die Reaktionszeit und die Hydroxylzahlen der Reaktionsprodukte aufgelistet. Desweiteren sind in den darauf folgenden Abbildungen der Beispiele 1 bis 6 die entsprechenden Produktverteilungen, die durch GC-Analyse erhalten wurden, dargestellt.

**Tabelle 1**

| Katalysator | c(Kat) Gew.-% | t h | OHZ | HLV | Abb. |
|---|---|---|---|---|---|
| Bsp. 1 | 0,5 | 0,75 | 174 | gut | 1 |
| Bsp. 2 | 0,5 | 1 | 170 | gut | 2 |
| Bsp. 3 | 0,5 | 0,8 | 173 | gut | 3 |
| Bsp. 4 | 0,5 | 2,75 | 176 | mäßig | 4 |
| Bsp. 5 | 0,5 | 3,5 | 178 | gut | 5 |
| Bsp. 6 | 0,5 | 2,3 | 178 | mäßig | 6 |
| Legende: c(Kat) = Katalysatorkonzentration t = Reaktionszeit OHZ = Hydroxylzahl HLV = Homologenverteilung | | | | | |

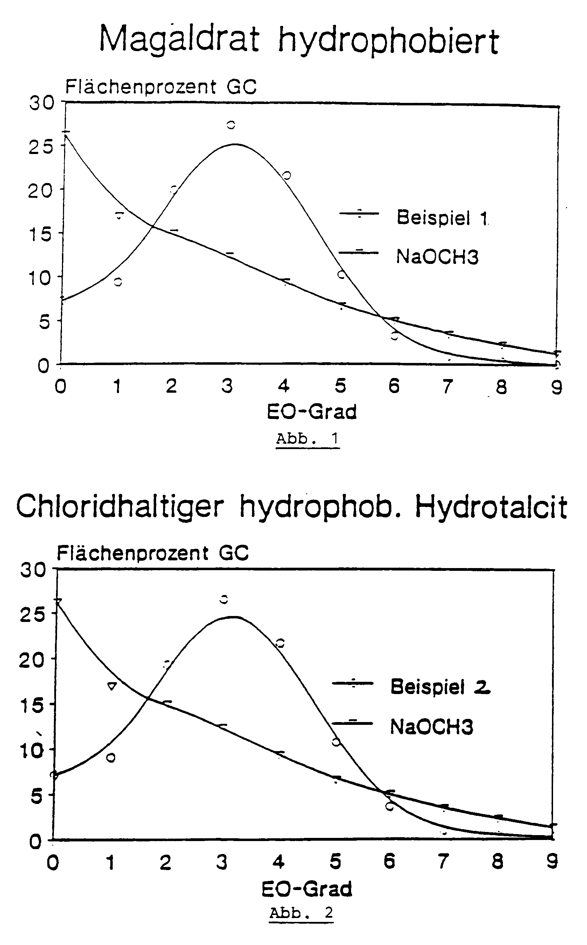

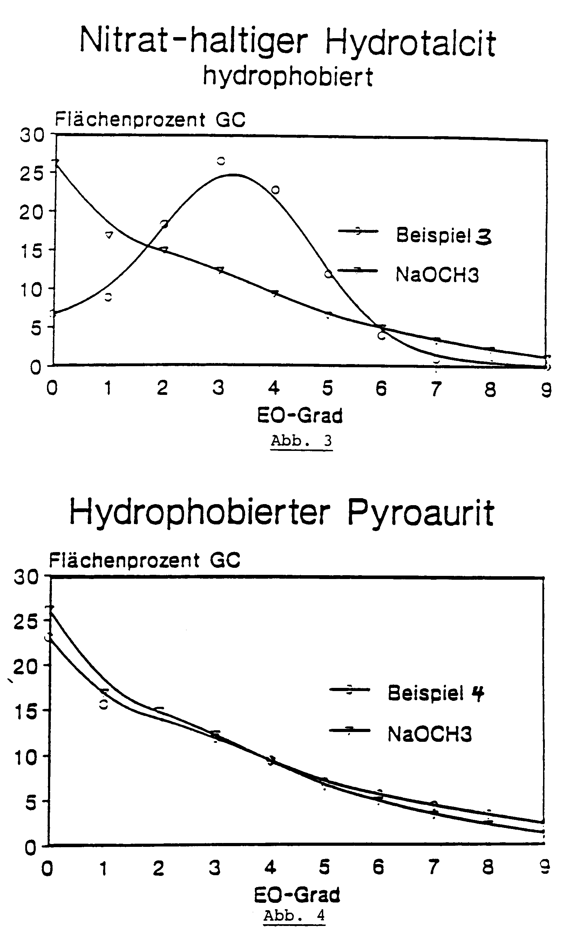

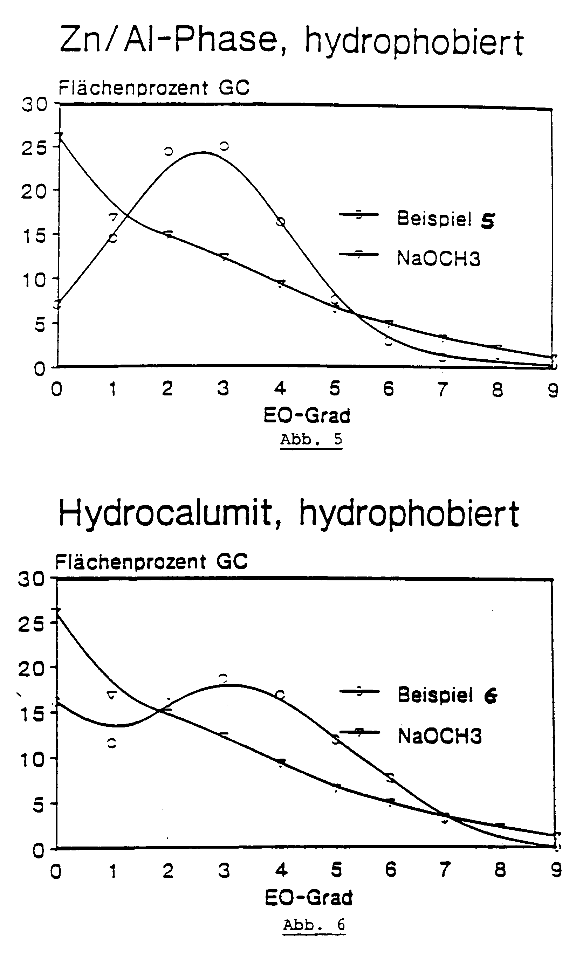

## Patentansprüche

1. Hydrophobierte Doppelschichthydroxid-Verbindungen der allgemeinen Formel **(I)**,
**[M(II)**_{**1-x**} **M(III)**_{**x**}**(OH)₂] A**_{**a**} **B**_{**b**} *** z H₂O (I)**
in der M(II) ein zweiwertiges Metallkation, ausgewählt aus der von Magnesium, Zink, Calcium, Eisen, Cobalt, Kupfer, Cadmium, Nickel und Mangan gebildeten Gruppe, bedeutet,
in der M(III) ein dreiwertiges Metallkation, ausgewählt aus der von Aluminium, Eisen, Chrom, Mangan, Wismut und Cer gebildeten Gruppe, bedeutet,
in der A für ein Äquivalent eines Monoanions einer aliphatischen Monocarbonsäure mit 2 bis 34 C-Atomen oder für ein Äquivalent eines Dianions einer aliphatischen Dicarbonsäure mit 4 bis 44 C-Atomen steht,
in der B ein Anion, aus der von Carbonat, Hydrogencarbonat, Sulfat, Nitrat, Nitrit, Phosphat, Hydroxid und Halogeniden gebildeten Gruppe, bedeutet,
und in der die Bedingungen
0,1 ≦ x ≦ 0,5
0 < a ≦ 0,5
0 ≦ b ≦ 0,5
0 < a + b ≦ 0,5
O ≦ z ≦ 10
gelten, wobei das Zahlenverhältnis von a zu b im Bereich von 0,49 : 0,01 bis 0,05 : 0,45 liegt und Verbindungen, die die Kombinationen von Magnesium und Aluminium mit Carbonat und/oder Sulfat enthalten, ausgeschlossen sind.

2. Doppelschichthydroxid-Verbindungen nach Anspruch 1, **dadurch gekennzeichnet**, daß in den hydrophobierten Doppelschichthydroxid-Verbindungen der allgemeinen Formel **(I)** das Zahlenverhältnis von a zu b im Bereich von 0,3 : 0,02 bis 0,05 : 0,25 liegt.

3. Doppelschichthydroxid-Verbindungen nach Anspruch 1 und 2, **dadurch gekennzeichnet**, daß die Anionen A von Monocarbonsäuren mit 6 bis 22 Kohlenstoffatomen abgeleitet sind.

4. Doppelschichthydroxid-Verbindungen nach Anspruch 3, **dadurch gekennzeichnet**, daß die hydrophobierten Doppelschichthydroxid-Verbindungen, bezogen auf ihr Gesamtgewicht 15 bis 70 Gew.-%, insbesondere 20 bis 50 Gew.-%, der Anionen A von Monocarbonsäuren mit 6 bis 22 Kohlenstoffatomen enthalten.

5. Doppelschichthydroxid-Verbindungen nach Anspruch 1 und 2, **dadurch gekennzeichnet**, daß die Dianionen von aliphatischen Dicarbonsäuren, einschließlich Dimerfettsäuren, mit 8 bis 36 Kohlenstoffatomen abgeleitet sind.

6. Doppelschichthydroxid-Verbindungen nach Anspruch 5, **dadurch gekennzeichnet**, daß die hydrophobierten Doppelschichthydroxid-Verbindungen, bezogen auf ihr Gesamtgewicht, 10 bis 60 Gew.-%, insbesondere 15 bis 50 Gew.-%, der Dianionen von aliphatischen Dicarbonsäuren, einschließlich Dimerfettsäuren, mit 8 bis 36 Kohlenstoffatomen enthalten.

7. Verfahren zur Herstellung von hydrophobierten Doppelschichthydroxid-Verbindungen der allgemeinen Formel **(I)**, **dadurch gekennzeichnet**, daß man Doppelschichthydroxid-Verbindungen der allgemeinen Formel **(II)**,
**[M(II)**_{**1-x**} **M(III)**_{**x**}**(OH)₂] B * z H₂O (II)**
in der M(II), M(III), B, x und z die in Anspruch 1 genannte Bedeutung besitzen, ausschließlich der Verbindungen, die die Kombination von Magnesium und Aluminium mit Carbonat und/oder Sulfat enthalten, mit wenigstens einer aliphatischen Monocarbonsäure mit 2 bis 34 C-Atomen und/oder wenigstens einer aliphatischen Dicarbonsäure mit 4 bis 44 C-Atomen entweder
a) in einem organischen Lösungsmittel umsetzt und das Lösungsmittel durch Trocknen bei 20 bis 150 °C entfernt oder
b) durch Rühren oder Kneten direkt miteinander umsetzt oder
c) die Doppelschichthydroxid-Verbindungen der allgemeinen Formel **(II)** mit einem Alkali- und/oder Erdalkalimetallsalz der Mono- und/oder Dicarbonsäuren in wäßriger Suspension umsetzt.

8. Verfahren nach Anspruch 7, **dadurch gekennzeichnet**, daß man bei der Umsetzung in einem organischen Lösungsmittel niedere Alkohole mit 1 bis 6 C-Atomen, vorzugsweise i-Propanol, offenkettige Ether, vorzugsweise Diethylether, cyclische Ether, vorzugsweise Tetrahydrofuran, und/oder Ketone, vorzugsweise Aceton, einsetzt.

9. Verfahren nach Anspruch 7 und 8, **dadurch gekennzeichnet**, daß man die Umsetzung bei einem Molverhältnis Doppelschichthydroxid-Verbindung zu Monocarbonsäure (bzw. Dicarbonsäure) von 6 : 1 (3 : 1) bis 1 : 10 (1 : 5), vorzugsweise 3 : 1 (1,5 : 1) bis 1 : 3 (1 : 1,5) bei Temperaturen von 20 bis 120 °C, vorzugsweise 40 bis 100 °C, durchführt.

10. Verfahren nach Anspruch 7 bis 9, **dadurch gekennzeichnet**, daß man das organische Lösungsmittel in 0,5 bis 3 Stunden, vorzugsweise 1 bis 2 Stunden, bei Temperaturen von 20 bis 150 °C, vorzugsweise 50 bis 120 °C, durch Trocknen entfernt.

11. Verwendung der hydrophobierten Doppelschichthydroxid-Verbindungen der allgemeinen Formel **(I)** nach Anspruch 1 bis 9, ausschließlich der Verbindungen, die die Kombination von Magnesium und Aluminium mit Carbonat enthalten, als Alkoxylierungskatalysatoren für Verbindungen mit aktiven H-Atomen oder für Fettsäureester.

12. Verwendung nach Anspruch 11, **dadurch gekennzeichnet**, daß die Verbindungen mit aktiven H-Atomen aus der von Fettsauren, Hydroxyfettsäuren, Fettsäureamiden, Alkanolen, Alkylphenolen, Polyglykolen, Fettaminen, Fettsäurealkanolamiden oder vicinal hydroxy- bzw. alkoxy-substituierten Alkanen gebildeten Gruppe ausgewählt sind.

13. Verwendung nach den Ansprüchen 11 und 12, **dadurch gekennzeichnet**, daß man die hydrophobierten Doppelschichthydroxid-Verbindungen in einer Menge von 0,1 bis 3 Gew.-%, vorzugsweise 0,5 bis 2 Gew.-%, bezogen auf das Endprodukt der Alkoxylierung, einsetzt.

## Claims

1. Hydrophobicized double layer hydroxide compounds corresponding to general formula **(I)**:
**[M(II)**_{**1-x**}**M(III)**_{**x**}**(OH)₂]A**_{**a**} **B**_{**b**} *** z H₂O (I)**
in which M(II) is a divalent metal cation selected from the group consisting of magnesium, zinc, calcium, iron, cobalt, copper, cadmium, nickel and manganese,
in which M(III) is a trivalent metal cation selected from the group consisting of aluminium, iron, chromium, manganese, bismuth and cerium,
in which A is an equivalent of a monoanion of an aliphatic C₂₋₃₄ monocarboxylic acid or an equivalent of a dianion of an aliphatic c₄₋₄₄ dicarboxylic acid,
in which B is an anion from the group consisting of carbonate, hydrogen carbonate, sulfate, nitrate, nitrite, phosphate, hydroxide and halides
and in which the conditions
0.1 ≦ x ≦ 0.5
0 < a ≦ 0.5
0 ≦ b ≦ 0.5
0 < a + b ≦ 0.5
0 ≦ z ≦ 10
apply, the number ratio of a to b being from 0.49:0.01 to 0.05:0.45 compounds containing the combinations of magnesium and aluminium with carbonate and/or sulfate being excluded.

2. Double layer hydroxide compounds as claimed in claim 1, **characterized in that** the number ratio of a to b in the hydrophobicized double layer hydroxide compounds of general formula **(I)** is in the range from 0.3:0.02 to 0.05:0.25.

3. Double hydroxide compounds as claimed in claims 1 and 2, **characterized in that** the anions A are derived from C₆₋₂₂ monocarboxylic acids.

4. Double hydroxide compounds as claimed in claim 3, **characterized in that** the hydrophobicized double layer hydroxide compounds, based on their total weight, contain 15 to 70% by weight and more particularly 20 to 50% by weight of the anions A of C₆₋₂₂ monocarboxylic acids.

5. Double layer hydroxide compounds as claimed in claims 1 and 2, **characterized in that** the dianions are derived from aliphatic C₈₋₃₆ dicarboxylic acids, including dimer fatty acids.

6. Double layer hydroxide compounds as claimed in claim 5, **characterized in that** the hydrophobicized double layer hydroxide compounds, based on their total weight, contain 10 to 60% by weight and more particularly 15 to 50% by weight of the dianions of aliphatic C₈₋₃₆ dicarboxylic acids, including dimer fatty acids.

7. A process for the production of hydrophobicized double layer hydroxide compounds corresponding to general formula **(I)**, **characterized in that** double layer hydroxide compounds corresponding to general formula **(II)**:
**[M(II)**_{**1-x**}**M(III)**_{**x**}**(OH)₂] B * z H₂O (II)**
in which M(II), M(III), B, x and z are as defined above, excluding compounds containing the combinations of magnesium and aluminium with carbonate and/or sulfate, are reacted with at least one aliphatic C₂₋₃₄ monocarboxylic acid and/or at least one C₄₋₄₄ aliphatic dicarboxylic acid either
a) in an organic solvent and the solvent is removed by drying at 20 to 150°C or
b) are directly reacted with one another by stirring or kneading or
c) the double layer hydroxide compounds corresponding to general formula **(II)** are reacted with an alkali metal and/or alkaline earth metal salt of the mono- and/or dicarboxylic acids in aqueous suspension.

8. A process as claimed in claim 7, **characterized in that** lower C₁₋₆ alcohols, preferably i-propanol, open-chain ethers, preferably diethyl ether, cyclic ethers, preferably tetrahydrofuran, and/or ketones, preferably acetone, are used where the reaction is carried out in an organic solvent.

9. A process as claimed in claims 7 and 8, **characterized in that** the reaction is carried out with a molar ratio of double layer hydroxide compound to monocarboxylic acid (or dicarboxylic acid) of 6:1 (3:1) to 1:10 (1:5), preferably 3:1 (1.5:1) to 1:3 (1:1.5) and at temperatures of 20 to 120°C and preferably 40 to 100°C.

10. A process as claimed in claims 7 to 9, **characterized in that** the organic solvent is removed by drying for 0.5 to 3 hours and preferably for 1 to 2 hours at temperatures of 20 to 150°C and preferably at temperatures of 50 to 120°C.

11. The use of the hydrophobicized double layer hydroxide compounds of general formula **(I)** according to claims 1 to 9, excluding the compounds containing a combination of magnesium and aluminium with carbonate, as alkoxylation catalysts for compounds containing active H atoms or for fatty acid esters.

12. The use claimed in claim 11, **characterized in that** the compounds containing active H atoms are selected from the group consisting of fatty acids, hydroxy-fatty acids, fatty acid amides, alkanols, alkyl phenols, polyglycols, fatty amines, fatty acid alkanolamides or vicinally hydroxy- or alkoxy-substituted alkanes.

13. The use claimed in claims 11 and 12, **characterized in that** the hydrophobicized double layer hydroxide compounds are used in a quantity of 0.1 to 3% by weight and preferably in a quantity of 0.5 to 2% by weight, based on the end product of the alkoxylation reaction.

## Revendications

1. Composés hydroxydes imperméabilisés à double couche de la formule générale (I)
[M(II)₁₋ₓM(III)ₓ(OH)₂]Aₐ B_{b} * z H₂O (I)
dans laquelle,
M(II) représente un cation métallique bivalent, choisi dans le groupe formé par le magnésium, zinc, calcium, fer, cobalt, cuivre, cadmium, nickel et manganèse.
M(III) représente un cation métallique trivalent, choisi dans le groupe formé par l'aluminium, fer, chrome, manganèse, bismuth et cérium.
A représente un équivalent d'un monoanion d'un acide monocarboxylique aliphatique ayant 2 à 34 atomes de C ou un équivalent d'un dianion d'un acide dicarboxylique aliphatique ayant 4 à 44 atomes de C.
B représente un anion du groupe formé par du carbonate, hydrocarbonate sulfate, nitrate, nitrite, phosphate, hydroxyde et halogénures, et sont valables les conditions :
0,1 ≦ x ≦ 0,5
0 < a ≦ 0,5
0 ≦ b ≦ 0,5
0 < a + b ≦ 0,5
0 ≦ z ≦ 10
pour lesquelles le rapport numérique de a à b se situe dans la zone de 0,49:0,01 à 0,05: 0,45 et les composés, qui contiennent les combinaisons de magnésium et aluminium avec du carbonate et/ou du sulfate, sont exclus.

2. Composés hydroxydes à double couche selon la revendication 1, caractérisés en ce que dans les composés hydroxydes imperméabilisés à double couche de la formule générale (I) le rapport numérique de a à b se situe dans la gamme de 0,3:0,02 à 0,05:0,25.

3. Composés hydroxydes à double couche selon les revendications 1 et 2, caractérisé en ce que, les anions A sont dérivés d'acides monocarboxyliques avec de 6 à 22 atomes de carbone.

4. Composés hydroxydes à double couche selon la revendication 3, caractérisés en ce que les composés hydroxydes à double couche imperméabilisés contiennent, par rapport à leur poids total de 15 à 70 % en poids, en particulier de 20 à 50 % en poids, des anions A d'acides monocarboxyliques ayant de 6 à 22 atomes de carbone.

5. Composés hydroxydes à double couche, selon les revendications 1 et 2, caractérisés en ce que les dianions sont dérivés d'acides carboxyliques aliphatiques, y compris les acides gras dimères, avec de 8 à 36 atomes de carbone.

6. Composés hydroxydes à double couche selon la revendication 5, caractérisés en ce que les composés hydroxydes à double couche imperméabilisés, par rapport à leur poids total, contiennent de 10 à 60 % en poids, en particulier de 15 à 50 % en poids, de dianions d'acides dicarboxyliques aliphatiques, y compris des acides gras dimères, avec de 8 à 36 atomes de carbone.

7. Procédé de préparation de composés hydroxydes à double couche imperméabilisés de la formule générale (I), caractérisé en ce qu'on transforme des composés hydroxydes à double couche de la formule générale (II),
[M(II)₁₋ₓM(III)ₓ(OH)₂]B * z H₂O (II)
dans laquelle M(II), M(III), B, x et z ont la signification indiquée ci-dessus, à l'exclusion des composés, qui contiennent la combinaison de magnésium et d'aluminium avec du carbonate et/ou du sulfate, avec au moins un acide monocarboxylique aliphatique ayant de 2 à 34 atomes de C et/ou au moins un acide dicarboxylique aliphatique ayant de 4 à 44 atomes de C, soit:
a) dans un solvant organique et on élimine le solvant par séchage de 20 à 150°C, soit,
b) par agitation ou malaxage directement entre eux, soit,
c) on transforme les composés hydroxydes à double couche de la formule générale (II) avec un sel de métal alcalin ou alcalino-terreux des acides mono et/ou dicarboxyliques, en suspension aqueuse.

8. Procédé selon la revendication 7, caractérisé en ce qu'on met en oeuvre lors de réaction dans un solvant organique des alcools inférieurs avec de 1 à 6 atomes de carbone, de préférence du i-propanol, des éthers à chaîne ouverte, de préférence des diéthyléther, des éthers cycliques, de préférence du tétrahydrofurane, et/ou des cétones, de préférence de l'acétone.

9. Procédé selon les revendications 7 et 8, caractérisé en ce qu'on réalise la réaction avec un rapport molaire du composé hydroxyde à double chaîne à l'acide monocarboxylique (ou de carboxylique) de 6:1 (3:1) à 1:10 (1:5), de préférence de 3:1 (1,5:1) à 1:3 (1:1,5) à des températures de 20 à 120°C, de préférence de 40 à 100°C.

10. Procédé selon les revendications 7 à 9, caractérisé en ce qu'on élimine le solvant organique par séchage en 0,5 à 3 heures, de préférence en 1 à 2 heures, à des températures de 20 à 150°C, de préférence de 50 à 120°C.

11. Utilisation des composés hydroxydes à double couche imperméabilisés de la formule générale (I) selon les revendications 1 à 9, à l'exclusion des composés, qui contiennent la combinaison de magnésium et d'aluminium avec du carbonate, comme catalyseurs d'alcoxylation pour des composés avec des atomes d'H actifs ou pour des esters d'acide gras.

12. Utilisation selon la revendication 11, caractérisée en ce que les composés avec des atomes H actifs sont choisis dans le groupe formé d'acides gras, d'hydroxy acides gras, d'amides d'acide gras, d'alcanols, d'alkylphénols, de polyglycols, d'amines grasses, d'amides d'alcanol d'acides gras ou d'alcanes substituées par un alcoxy.

13. Utilisation selon les revendications 11 et 12, caractérisée en ce qu'on met en oeuvre les composés hydroxydes à double couche imperméabilisés en une quantité de 0,1 à 3 % en poids, de préférence de 0,5 à 2 % en poids, par rapport au produit final de l'alcoxylation.
